(19) Europäisches Patentamt
European Patent Office
Office européen des brevets

(11) EP 1 077 917 B1

(12) **EUROPÄISCHE PATENTSCHRIFT**

(45) Veröffentlichungstag und Bekanntmachung des Hinweises auf die Patenterteilung:
**01.10.2003 Patentblatt 2003/40**

(21) Anmeldenummer: **99920861.4**

(22) Anmeldetag: **10.05.1999**

(51) Int Cl.$^7$: **C07C 29/70**

(86) Internationale Anmeldenummer:
**PCT/EP99/03188**

(87) Internationale Veröffentlichungsnummer:
**WO 99/058482 (18.11.1999 Gazette 1999/46)**

(54) **HERSTELLUNG VON ALKALIMETALLALKOHOLATEN**

METHOD FOR PRODUCING ALKALI METAL ALCOHOLATES

PREPARATION D'ALCOOLATES DE METAL ALCALIN

(84) Benannte Vertragsstaaten:
**AT CH DE FR GB IE IT LI**

(30) Priorität: **13.05.1998 DE 19821304**

(43) Veröffentlichungstag der Anmeldung:
**28.02.2001 Patentblatt 2001/09**

(73) Patentinhaber: **Chemetall GmbH**
**60487 Frankfurt am Main (DE)**

(72) Erfinder:
- **WIETELMANN, Ulrich**
**D-61381 Friedrichsdorf (DE)**
- **LISCHKA, Uwe**
**D-60437 Frankfurt am Main (DE)**
- **EMMEL, Ute**
**D-65929 Frankfurt am Main (DE)**

(74) Vertreter: **Uppena, Franz, Dr. et al**
**Dynamit Nobel Aktiengesellschaft,**
**Patente, Marken & Lizenzen**
**53839 Troisdorf (DE)**

(56) Entgegenhaltungen:
**EP-A- 0 810 193**
**DE-A- 3 437 152**
**DE-C- 845 341**
**FR-A- 1 070 601**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 077 917 B1

Printed by Jouve, 75001 PARIS (FR)

## Beschreibung

Die Erfindung betrifft ein Verfahren zur Herstellung von Alkalimetallalkoholaten, bei dem Alkohol mit Alkalimetall in einem aprotischen organischen Lösungsmittel unter Verwendung eines Wasserstoffakzeptors umgesetzt wird.
Alkalimetallalkoholate R-OM (R = Alkyl, M = Li, Na, K, Rb, Cs) sind hydrolyseempfindliche Verbindungen, die wegen ihrer basischen Eigenschaften in der organischen Synthese häufig Verwendung finden.
Es ist bekannt, Alkalimetallalkoholate durch Umsetzung der entsprechenden Alkohole mit einem Alkalimetall herzustellen gemäß der Reaktionsgleichung:

$$2\ \text{R-OH} + 2\ \text{M} \rightarrow 2\ \text{R-OM} + \text{H}_2$$

R = Alkyl
M = Li, Na, K, Rb, Cs

Die Geschwindigkeit dieser Umsetzung verringert sich mit steigender Länge der Alkylkette sowie mit zunehmender Verzweigung. Während es im Laboratoriumsmaßstab gelingt, die Umsetzung durch Einsatz von äußerst feinteiligem Alkalimetall, welches mittels Hochgeschwindigkeitsrührer mit einer Teilchengröße unter 50 µm erzeugt wird, erheblich zu beschleunigen, benötigt die Umsetzung im technischen Produktionsmaßstab viele Stunden. Lange Reaktionszeiten beeinträchtigen jedoch die Wirtschaftlichkeit dieser Alkoholatsynthese.
Aus der FR-PS 1 070 601 ist ein Verfahren zur Herstellung von Alkalimetallalkoholaten bekannt, wobei Alkalimetall unter Rühren in siedendem, inertem Kohlenwasserstoff fein verteilt wird und nach dem Erkalten der Dispersion die berechnete Menge Alkohol zugetropft wird. Bei der Herstellung der Natriumsuspension wird eine Agglomeration des fein verteilten Natriums durch die Zugabe eines dispergierenden Zusatzstoffes, wie Fettsäure, Tenside oder Aktivkohle, verhindert. Das in Xylol ausfallende Alkalimetallalkoholat kann abgetrennt werden.
Bei dem aus der DE-OS 34 37 152 bekannten Verfahren zur katalysierten Herstellung von Alkalimetallalkoholaten aus Alkaliamalgamen und Alkoholen wird stückiger Anthrazit als Katalysator eingesetzt, dessen Oberfläche vorzugsweise mit einer Mischung aus Nickel- und Molybdänoxid belegt ist. Bevorzugt werden aliphatische Alkohole mit 1 bis 4 Kohlenstoffatomen verwendet.
Bei dem aus der DE-PS 08 45 341 bekannten Verfahren zur Herstellung ätzalkaliarmer Alkalimetallalkoholate wird amalgamiertes Alkalimetall in Gegenwart von Katalysatoren, wie Graphit, mehrmals mit Alkohol in Berührung gebracht. Hierzu ist es des weiteren aus DE-PS 09 28 467 bekannt, feinverteiltes Natriumamalgam und Alkohol im Gegenstrom über einen stückigen Katalysator, bestehend aus einer Mischung aus Graphit oder Aktivkohle und Metallspänen, zu leiten.
Die oben aufgeführten Verfahren weisen folgende Nachteile auf:

- Durch den Einsatz stückiger bzw. spanförmiger Katalysatorstoffe im technischen Produktionsmaßstab gelingt es nicht, die Reaktionszeiten in wirtschaftlicher Weise zu senken.
- Die erforderliche Abtrennung der Katalysatoren vom Reaktionsprodukt ist in vielen Fällen problematisch.
- Die Verwendung einer toxischen Amalgamverbindung als Alkalimetallkomponente ist wegen der Arbeitsplatz- und Umweltbelastung problematisch.

Da die Reaktionsgeschwindigkeit bei der Verwendung von elementarem Alkalimetall besonders im Fall sterisch gehinderter tertiärer Alkohole trotz der beschriebenen Maßnahmen häufig nur unbefriedigend ist, werden auch stark basische Organometallverbindungen als Alkalimetallquelle eingesetzt. Dies gilt insbesondere für die Herstellung von Lithiumalkoholaten:

$$\text{R-OH} + \text{R'Li} \rightarrow \text{R-OLi} + \text{R'H} \uparrow$$

Nachteil dieser glatt ablaufenden Reaktion ist der relativ hohe Preis von Organolithiumverbindungen.
Weitere Synthesen gehen von Alkalimetallhydriden und - amiden aus. Diese Reagenzien reagieren zwar häufig etwas oder deutlich schneller als das Alkalimetall in elementarer Form, jedoch sind die Verbindungen, auf Molbasis gerechnet, deutlich teurer als das Alkalimetall. Außerdem entsteht im Fall der Amide Ammoniak, das kostenaufwendig aus dem Abgasstrom entfernt werden muß. Beim Einsatz von Hydriden entsteht - verglichen mit dem Einsatz der elementaren Metalle - die doppelte Wasserstoffmenge. Wasserstoff ist zwar kein ökologisch bedenkliches Produkt, jedoch ist der entstehende Gasstrom mit organischen Verbindungen (Lösungsmittel, Alkohol) belastet, die aus ökologischen Gründen möglichst nicht in die Umwelt gelangen sollten.

$$R\text{-}OH + MH \rightarrow R\text{-}OM + H_2 \uparrow$$

$$R\text{-}OH + MNH_2 \rightarrow R\text{-}OM + NH_3 \uparrow$$

R = Alkylrest; M = Alkalimetall

Aufgabe der vorliegenden Erfindung ist es, die dem Stand der Technik entsprechenden Nachteile zu vermeiden, d.h., insbesondere ein Verfahren zur Herstellung von Alkalimetallalkoholaten aufzuzeigen, das
von billigen, technisch verfügbaren Rohstoffen ausgeht, in einer schnellen Reaktion
unter Bildung möglichst weniger gasförmiger Nebenprodukte
ohne Benutzung schlecht abtrennbarer fester Katalysatoren
wasserfreie Alkalimetallalkoholate liefert.

Die Aufgabe wird dadurch gelöst, daß der Alkohol mit dem Alkalimetall (Li, Na, K, Rb, Cs) in einem aprotischen organischen Lösungsmittel umgesetzt wird und dabei ein H-Akzeptor in Form eines konjugierten Diens oder eines 1-Arylolefins zugesetzt wird. Die Reaktion verläuft dabei bevorzugt nach folgendem Reaktionsschema:

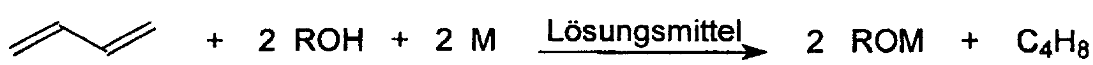


bzw.

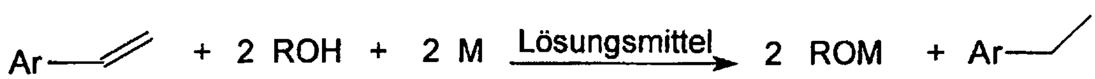


Ar = Arylrest

Die Gegenwart eines H-Akzeptors bewirkt eine unter Reaktionsführungs- und Umweltschutzgesichtspunkten vorteilhafte Reduktion der Abgasmenge verglichen mit der herkömmlichen Alkalimetallalkoholatbildungsreaktion.

Als H-Akzeptoren können offenkettige oder zyklische, nicht substituierte oder substituierte 1,3-Diene

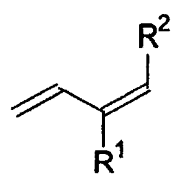

mit $R_1$, $R_2$ = H, Alkyl, Vinyl
($R_1$, $R_2$ in cis- oder trans-Stellung),

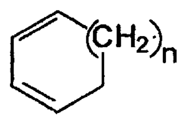

mit n = 1 bis 5
oder nicht substituierte oder substituierte 1-Arylolefine

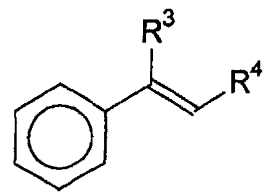

mit $R_3$, $R_4$ = H, Alkyl
($R_3$, $R_4$ in cis- oder trans-Stellung)
eingesetzt werden (im Falle der substituierten Reagenzien sowohl in der cis- wie in der trans-Form). Bevorzugte H-Akzeptoren für diese Reaktion sind Isopren, Butadien, Cyclohexadien-(1,3), Styrol oder Methylstyrol.

Die Zugabemenge des H-Akzeptors beträgt das 0,2 bis 4-fache, bevorzugt das 0,4 bis 1,5-fache der stöchiometrischen Menge, d.h. 0,2 bis 4 Mol, bevorzugt 0,4 bis 1,5 Mol, bezogen auf jeweils 2 Mol Alkohol. Das Verfahren läßt sich somit auch mit einer unter der Stöchiometrie liegenden Zugabemenge des H-Akzeptors durchführen, was die Wirtschaftlichkeit erhöht.

Als Alkalimetall kann insbesondere eines der Metalle Li, Na oder K oder Mischungen dieser Metalle eingesetzt werden.

Von Vorteil ist, daß das Alkalimetall in pulvriger, granulierter oder grobstückiger Form vorliegen kann. Im Falle von Na, K, Rb oder Cs kann bevorzugt auch eine feinverteilte Schmelze gewählt werden. Aufgrund seines hohen Schmelzpunktes wird das Lithium bevorzugt in fester Form eingesetzt.

Insbesondere bei der Umsetzung von sekundären oder tertiären Alkoholen mit einem Alkalimetall führt die Gegenwart eines H-Akzeptors zu deutlich höheren Reaktionsgeschwindigkeiten relativ zu bisher bekannten Verfahren. Von besonderem kommerziellen Interesse sind die Reaktionen mit i-Propanol, t-Butanol oder t-Pentanol.

Als aprotisches organisches Lösungsmittel kann ein aliphatischer oder aromatischer Kohlenwasserstoff mit 4 bis 20 C-Atomen oder ein Ether oder ein Gemisch dieser Stoffe eingesetzt werden. Besonders gut läßt sich die Reaktion in Hexan, Heptan, Octan, Toluol, Ethylbenzol, Methyl-tert.-Butylether (MTBE), Tetrahydrofuran (THF) oder 2-Methyl-THF durchführen. Auch kommerziell verfügbare Kohlenwasserstoffgemische, wie z.B. Petrolether, Paraffinöl, hochsiedendes Shellsol D 70, können besonders gut als Lösungsmittel dienen.

Vorzugsweise wird zur Dispersion von Alkalimetall im aprotischen organischen Lösungsmittel das Gemisch aus Alkohol und H-Akzeptor zugegeben. Möglich ist auch das Vorlegen eines Gemisches aus Lösungsmittel, H-Akzeptor und Metall, zu dem der Alkohol zudosiert wird. In einigen Fällen ist es auch möglich, das Alkalimetall in fester oder flüssiger Form zur Mischung aus Lösungsmittel, Alkohol und H-Akzeptor zuzugeben.

Beispielsweise kann auf diese Weise eine Lösung von Lithium tert-butylat in THF hergestellt werden.

Die Reaktionstemperatur wird bei -20 bis 200°C, bevorzugt bei 20 bis 140°C gehalten.

Das Verfahren kann so durchgeführt werden, daß evtl. überschüssiges Alkalimetall abfiltriert wird und als Endprodukt eine Alkalimetallalkoholatlösung erhalten wird.

Das Verfahren kann weiterhin so durchgeführt werden, daß nach Beendigung der Reaktion evtl. überschüssiges Alkalimetall abfiltriert wird, die Reaktionslösung eingedampft und als Endprodukt festes Alkalimetallalkoholat erhalten wird.

Der Gegenstand der Erfindung wird nachfolgend anhand von Ausführungsbeispielen näher erläutert.

**Beispiel 1: Synthese von Natrium tert-butylat (NTB) in Toluol in Gegenwart einer stöchiometrischen Menge Styrol**

In einem 250-ml-Vierhalskolben mit Heizpilz, KPG-Rührer und Rückflußkühler wurden 4,78 g (208 mmol) Na-Stücke in 69,8 g Toluol vorgelegt und auf ca. 100°C aufgeheizt. Beim Beginn der Zugabe eines Gemisches aus 15,0 g (202 mmol) tert-Butanol und 10,5 g (101 mmol) Styrol erhitzte sich das Reaktionsgemisch sofort auf 109°C. Während der Zugabe, die in 25 Minuten erfolgte, war keine signifikante Gasentwicklung zu beobachten. Gegen Ende der Zugabe waren an der Kolbenwandung kleine Mengen eines weißen Niederschlages zu beobachten, der jedoch 5 Minuten nach Dosierungsende verschwand (klare, hellgelbe Lösung). Die Temperatur wurde noch 25 Minuten bei etwa 100°C gehalten.

Die Produktlösung wurde heiß filtriert und im Rotationsverdampfer bis zur Gewichtskonstanz getrocknet. Es wurden 18,2 g (94 %) NTB in Form eines farblosen Pulvers erhalten.

**Vergleichsbeispiel A: Synthese von Natrium tert-butylat in Toluol ohne H-Akzeptor**

In der gleichen Apparatur wie in Beispiel 1 wurde tert-Butanol in eine siedende toluolische Na-Dispersion (enthaltend 61,5 g Natrium = 2,7 mol) eindosiert. Nach Zugabe von 70 g tert-Butanol (35 Mol%, bezogen auf die eingesetzte Na-Menge) hatten sich nach 1,4 Stunden erst 17 % der theoretisch zu erwartenden Menge Wasserstoff gebildet, was auf eine im Vergleich zum erfindungsgemäßen Beispiel 1 wesentlich geringere Reaktionsgeschwindigkeit schließen läßt.

**Beispiel 2: Synthese von Lithium tert-butylat in Tetrahydrofuran in Gegenwart einer stöchiometrischen Menge Isopren**

In einem 1-l-Doppelmantelreaktor mit Rückflußkühler, Tropftrichter und KPG-Rührer wurden 9,62 g (1386 mmol) Lithiumgranulat (Na-Gehalt 0,43 %) in 400 g THF vorgelegt und bei 15 bis 35°C mit einem Gemisch aus 106 g (1430 mmol) tert-Butanol und 47,2 g (693 mmol) Isopren versetzt. Die Reaktion sprang sofort zu Beginn der 45minütigen Dosierzeit an; es mußte kräftig gekühlt werden. Nach einer 30minütigen Nachreaktionszeit war nur noch eine sehr kleine Menge Li-metall in einer ansonsten klaren, leicht graugefärbten Produktlösung vorhanden. In einer Lösungsprobe wurden 2,35 mmol/g Gesamtbasekonzentration nachgewissen (entsprechend einem 95%igen Umsatz). Nach einer weiteren Stunde bei 30°C stieg die Basenkonzentration auf 2,43 mmol/g (entspr. 98 %). Während der gesamten Reaktionszeit wurde keine signifikante Wasserstoffentwicklung beobachtet.

Die Produktlösung ließ sich ohne Probleme filtrieren (Glasfritte, Filtrationszeit ca. 1 Minute).

**Vergleichsbeispiel B: Synthese von Lithium tert-butylat in Tetrahydrofuran ohne H-Akzeptor**

In einem 500-ml-Kolben mit Rückflußkühler, Tropftrichter und KPG-Rührer wurden 3,5 g (0,5 mol) Li-Granulat (0,77 % Na-Gehalt) in 100 g THF suspendiert und unter Rückfluß mit einer Lösung von 37,1 g (0,5mol) tert-Butanol in 60 ml THF innerhalb von 145 Minuten versetzt. Während dieser Zeit wurden 28 % der theoretisch zu erwartenden $H_2$-Menge gebildet. Nach weiteren 3 Stunden Kochen am Rückfluß wurde eine filtrierte Probe genommen und auf Gesamtbase untersucht (2,04 mmol/g entspr. 82 % Umsatz).

Nach Abkühlung wurde über eine Glasfritte filtriert. Der Filtrationsvorgang nahm 100 Minuten in Anspruch und lieferte eine trübe, gelbe Lösung. Die Reaktionszeit ist auch in diesem Vergleichsbeispiel gegenüber Beispiel 2 erhöht.

**Beispiel 3: Synthese von Kalium tert-amylat (KTA) in Hexan bei 60°C in Gegenwart einer stöchiometrischen Menge Isopren**

In einem 0,5-l-Doppelmantelreaktor mit KPG-Glasrührer, Rückflußkühler und Tropftrichter wurden 25,3 g (648 mmol) von Krusten gesäubertes Kalium (Firma Merck) in 305 g Hexan aufgeschmolzen und dann bei ca. 60°C mit einer Mischung aus 57,1 g (648 mmol) tert-Amylalkohol und 22,1 g (324 mmol) Isopren innerhalb 100 Minuten versetzt. Während des Zugabevorgangs wurde das Reaktionsgemisch leicht trüb, es war aber gut zu rühren.
Nach beendeter Zugabe wurde 10 Minuten refluxiert, wobei sich eine klare, leicht gelbliche Lösung bildete.

Die filtrierte Lösung wurde im Rotationsverdampfer im Vakuum eingedampft. Es wurden 74,8 g (92 %) eines farblosen Pulvers erhalten, das die für KTA erwartete Zusammensetzung aufwies.

**Beispiel 4: Synthese von Lithium tert-butylat in THF in Gegenwart einer unterstöchiometrischen Menge Isopren**

In einer Apparatur wie in Beispiel 3 wurden 5,2 g (0,75 mol) Lithiummetallgranulat (0,4% Na-Gehalt) in 300 ml THF suspendiert und innerhalb von 3 Stunden mit einem Gemisch aus 16,2 g (238 mmol) Isopren und 57,3 g (772 mmol) tert-Butanol versetzt. Die Reaktionstemperatur wurde bei ca. 40°C gehalten. Nach Dosierungsende wurde noch ca. 1 Stunde bei 40°C nachgerührt; danach war kein metallisches Lithium mehr zu erkennen. Die leicht trübe Lösung wurde ohne Schwierigkeiten über eine Glasfritte filtriert (Filtrationszeit ca. 30 sec). Im leicht grau gefärbten, aber klaren Filtrat wurde eine Basekonzentration von 2,18 mmol/g analysiert, was einer Ausbeute von 98% entspricht.

Während der Reaktionsphase hatten sich ca. 170 mmol $H_2$-Gas gebildet, d.h. ca. 45% des Metalls haben direkt mit dem Alkohol reagiert. Die eingesetzte Menge Isopren entsprach bei diesem Beispiel der 0,62-fachen stöchiometrischen Menge.

**Patentansprüche**

1. Verfahren zur Herstellung von Alkalimetallalkoholaten durch Umsetzung von Alkohol mit Alkalimetall in einem aprotischen organischen Lösungsmittel, **dadurch gekennzeichnet, daß** die Synthese in Gegenwart eines H-Akzeptors durchgeführt wird, wobei der H-Akzeptor ein offenkettiges, nicht substituiertes oder substituiertes 1,3-Dien

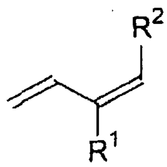

mit $R_1$, $R_2$ = H, Alkyl, Vinyl
($R_1$, $R_2$ in cis- oder trans-Stellung)
oder ein zyklisches 1,3-Dien

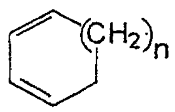

mit n = 1 bis 5
oder ein nicht substituiertes oder substituiertes1-Arylolefin

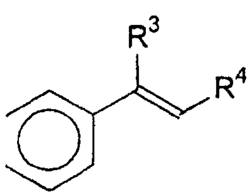

mit $R_3$, $R_4$ = H, Alkyl
($R_3$, $R_4$ in cis- oder trans-Stellung)
ist.

2. Verfahren nach Anspruch 1, **dadurch gekennzeichnet, daß** der H-Akzeptor Isopren, Butadien, Cyclohexadien-1,3; Styrol oder Methylstyrol ist.

3. Verfahren nach den Ansprüchen 1 oder 2, **dadurch gekennzeichnet, daß** der H-Akzeptor in einer Menge von 0,2 bis 4 Mol jeweils pro 2 Mol Alkohol eingesetzt wird.

4. Verfahren nach Anspruch 3, **dadurch gekennzeichnet, daß** der H-Akzeptor in einer Menge von 0,4 bis 1,5 Mol jeweils pro 2 Mol Alkohol eingesetzt wird.

5. Verfahren nach den Ansprüchen 1 bis 4, **dadurch gekennzeichnet, daß** als Alkalimetall eines oder mehrere der Metalle Li, Na oder K eingesetzt werden.

6. Verfahren nach den Ansprüchen 1 bis 5, **dadurch gekennzeichnet, daß** das Alkalimetall in fester Form (pulvrig, granuliert oder grobstückig) oder im Falle des Na, K, Rb oder Cs auch in feinverteilter, flüssiger Form vorliegt.

7. Verfahren nach den Ansprüchen 1 bis 6, **dadurch gekennzeichnet, daß** als Alkohol ein sekundärer oder tertiärer

Alkohol eingesetzt wird.

**8.** Verfahren nach Anspruch 7, **dadurch gekennzeichnet, daß** als Alkohol i-Propanol oder t-Butanol oder t-Pentanol eingesetzt wird.

**9.** Verfahren nach den Ansprüchen 1 bis 8, **dadurch gekennzeichnet, daß** als aprotisches Lösungsmittel ein aliphatischer oder aromatischer $C_4$- bis $C_{20}$-Kohlenwasserstoff oder ein Ether oder ein Gemisch der genannten Stoffe eingesetzt wird.

**10.** Verfahren nach Anspruch 9, **dadurch gekennzeichnet, daß** als aprotisches Lösungsmittel Hexan oder Heptan oder Cctan oder Toluol oder Ethylbenzol oder Methyl-tert.-Butylether oder Diethylether oder Tetrahydrofuran oder 2-Methyltetrahydrofuran eingesetzt wird.

**11.** Verfahren nach Anspruch 9, **dadurch gekennzeichnet, daß** als aprotisches Lösungsmittel ein Kohlenwasserstoffgemisch, wie Petrolether oder hochsiedende Kohlenwasserstoffmischungen (Paraffinöl), eingesetzt wird.

**12.** Verfahren nach den Ansprüchen 1 bis 11, **dadurch gekennzeichnet, daß** zur Alkalimetalldispersion in einem Lösungsmittel ein Gemisch aus Alkohol und H-Akzeptor gegeben wird.

**13.** Verfahren nach den Ansprüchen 1 bis 12, **dadurch gekennzeichnet, daß** die Reaktionstemperatur bei -20 bis 200°C gehalten wird.

**14.** Verfahren nach Anspruch 13, **dadurch gekennzeichnet, daß** die Reaktionstemperatur bei 20 bis 140°C gehalten wird.

**15.** Verfahren nach den Ansprüchen 1 bis 14, **dadurch gekennzeichnet, daß** evtl. überschüssiges Alkalimetall abfiltriert wird und als Endprodukt eine Alkalimetallalkoholatlösung erhalten wird.

**16.** Verfahren nach den Ansprüchen 1 bis 14, **dadurch gekennzeichnet, daß** nach Beendigung der Reaktion evtl. überschüssiges Alkalimetall abfiltriert wird, die Reaktionslösung eingedampft und als Endprodukt festes Alkalimetallalkoholat erhalten wird.

## Claims

**1.** Method for the preparation of alkali metal alcoholates by reacting alcohol with alkali metal in an aprotic organic solvent, **characterised in that** the synthesis is carried out in the presence of an H-acceptor, with the H-acceptor being an open-chain, unsubstituted or substituted 1,3-diene

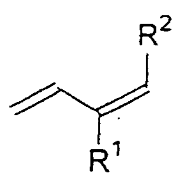

with $R_1$, $R_2$ = H, alkyl, vinyl
($R_1$, $R_2$ in cis or trans position)
or a cyclic 1,3-diene

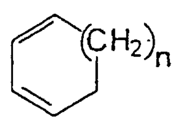

with n = 1 to 5
or an unsubstituted or substituted arylolefin

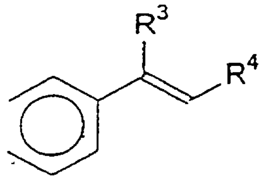

with $R_3$, $R_4$ = H, alkyl
($R_3$, $R_4$ in cis or trans position).

**2.** Method according to claim 1, **characterised in that** the H-acceptor is isoprene, butadiene, cyclohexadiene-1,3; styrene or methyl styrene.

**3.** Method according to claims 1 or 2, **characterised in that** the quantity of H-acceptor used is 0.2 to 4 mol per 2 mol alcohol.

**4.** Method according to claim 3, **characterised in that** the quantity of H-acceptor used is 0.4 to 1.5 mol per 2 mol alcohol.

**5.** Method according to claims 1 to 4, **characterised in that** one or more of the metals Li, Na or K is or are used as the alkali metal.

**6.** Method according to claims 1 to 5, **characterised in that** the alkali metal is present in solid form (pulverulent form, granular form or in lumps) or, in the case of the Na, K, Rb or Cs, even in a finely divided, liquid form.

**7.** Method according to claims 1 to 6, **characterised in that** a secondary or tertiary alcohol is used as the alcohol.

**8.** Method according to claim 7, **characterised in that** i-propanol or t-butanol or t-pentanol is used as the alcohol.

**9.** Method according to claims 1 to 8, **characterised in that** an aliphatic or aromatic $C_4$- to $C_{20}$-hydrocarbon or an ether or a mixture of the substances mentioned is used as the aprotic solvent.

**10.** Method according to claim 9, **characterised in that** hexane or heptane or octane or toluene or ethyl benzene or methyl tert.-butyl ether or diethyl ether or tetrahydrofuran or 2-methyl tetrahydrofuran is used as the aprotic solvent.

**11.** Method according to claim 9, **characterised in that** a hydrocarbon mixture, such as petroleum ether or high-boiling hydrocarbon mixtures (paraffin oil), is used as the aprotic solvent.

**12.** Method according to claims 1 to 11, **characterised in that** a mixture of alcohol and H-acceptor is added to the alkali-metal dispersion in a solvent.

**13.** Method according to claims 1 to 12, **characterised in that** the reaction temperature is maintained at -20 to 200°C.

**14.** Method according to claim 13, **characterised in that** the reaction temperature is maintained at 20 to 140°C.

**15.** Method according to claims 1 to 14, **characterised in that** possible excess alkali metal is filtered off, and an alkali metal alcoholate solution is obtained as an end product.

**16.** Method according to claims 1 to 14, **characterised in that** at the end of the reaction possible excess alkali metal is filtered off, the reaction solution is concentrated by evaporation, and solid alkali metal alcoholate is obtained as an end product.

## Revendications

1. Procédé de préparation d'alcoolates de métal alcalin par réaction d'un alcool avec un métal alcalin dans un solvant organique aprotique, **caractérisé en ce que** l'on réalise la synthèse en présence d'un accepteur d'hydrogène qui est un diène-1,3 à chaîne ouverte, non-substitué ou substitué, de formule :

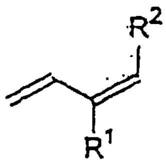

dans laquelle $R^1$ et $R^2$ représentent chacun un atome d'hydrogène, un groupe alkyle ou un groupe vinyle ($R^1$ et $R^2$ peuvent être en position cis ou trans),
ou un diène-1,3 cyclique de formule :

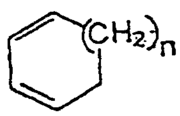

dans laquelle n désigne un nombre entier de 1 à 5,
ou encore une aryloléfine non-substituée ou substituée, de formule :

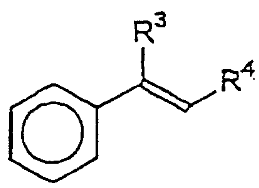

dans laquelle $R^3$ et $R^4$ représentent chacun un atome d'hydrogène ou un groupe alkyle ($R^3$ et $R^4$ peuvent être en position cis ou trans).

2. Procédé selon la revendication 1, **caractérisé en ce que** l'accepteur d'hydrogène est l'isoprène, le butadiène, le cyclohexadiène-1,3, le styrène ou le méthylstyrène.

3. Procédé selon la revendication 1 ou 2, **caractérisé en ce que** l'accepteur d'hydrogène est utilisé en une quantité de 0,2 à 4 moles pour 2 moles d'alcool.

4. Procédé selon la revendication 3, **caractérisé en ce que** l'accepteur d'hydrogène est utilisé en une quantité de 0,4 à 1,5 mole pour 2 moles d'alcool.

5. Procédé selon l'une quelconque des revendications 1 à 4, **caractérisé en ce qu'**on utilise comme métal alcalin un ou plusieurs des métaux Li, Na et K.

6. Procédé selon l'une quelconque des revendications 1 à 5, **caractérisé en ce que** le métal alcalin se présente sous une forme solide (pulvérulente, en granules ou en gros morceaux) ou également, dans le cas du sodium, du potassium, du rubidium ou du césium, sous une forme liquide, finement divisée.

7. Procédé selon l'une quelconque des revendications 1 à 6, **caractérisé en ce que** l'alcool utilisé est un alcool secondaire ou tertiaire.

**8.** Procédé selon la revendication 7, **caractérisé en ce que** l'alcool utilisé est l'isopropanol, le t-butanol ou le t-pentanol.

**9.** Procédé selon l'une quelconque des revendications 1 à 8, **caractérisé en ce que** l'on utilise comme solvant aprotique un hydrocarbure aliphatique ou aromatique, en $C_4$ à $C_{20}$, ou un éther ou un mélange de ces produits.

**10.** Procédé selon la revendication 9, **caractérisé en ce que** l'on utilise comme solvant aprotique, l'hexane, l'heptane, l'octane, le toluène, l'éthylbenzène, l'oxyde de méthyle et de tert.-butyle, l'oxyde diéthylique, le tétrahydrofurane ou le 2-méthyltétrahydrofurane.

**11.** Procédé selon la revendication 9, **caractérisé en ce que** l'on utilise comme solvant aprotique, un mélange d'hydrocarbures, comme l'éther de pétrole ou des mélanges d'hydrocarbures à point d'ébullition élevé (huile de paraffine).

**12.** Procédé selon l'une quelconque des revendications 1 à 11, **caractérisé en ce que** l'on ajoute un mélange d'alcool et d'accepteur d'hydrogène à la dispersion de métal alcalin dans un solvant.

**13.** Procédé selon l'une quelconque des revendications 1 à 12, **caractérisé en ce que** la température de réaction est maintenue à une valeur comprise entre -20°C et 200°C.

**14.** Procédé selon la revendication 13, **caractérisé en ce que** la température de réaction est maintenue à une valeur comprise entre 20°C et 140°C.

**15.** Procédé selon l'une quelconque des revendications 1 à 14, **caractérisé en ce que** le métal alcalin éventuellement en excès est séparé par filtration et on obtient comme produit final une solution d'alcoolate de métal alcalin.

**16.** Procédé selon l'une quelconque des revendications 1 à 14, **caractérisé en ce que**, une fois terminée la réaction, on sépare par filtration le métal alcalin éventuellement en excès, on évapore la solution réactionnelle et on obtient comme produit final un alcoolate de métal alcalin solide.